# EUROPEAN PATENT SPECIFICATION

(11) **EP 2 945 535 B1**
(45) Date of publication and mention of the grant of the patent: **25.03.2020**
(21) Application number: 14701326.2
(22) Date of filing: 17.01.2014
(51) Int. Cl.: A61B 5/0484, A61B 5/00

(54) **METHOD AND SYSTEM FOR MONITORING DEPTH OF ANAESTHESIA AND SENSORY FUNCTIONING**
VERFAHREN UND SYSTEM ZUR ÜBERWACHUNG DER NARKOSETIEFE UND SENSORISCHEN FUNKTION
PROCÉDÉ ET SYSTÈME DE SURVEILLANCE DE PROFONDEUR D'ANESTHÉSIE ET DE FONCTIONNEMENT SENSORIEL

(30) Priority: 17.01.2013 US 201361753883 P
(43) Date of publication of application: 25.11.2015
(73) Proprietor: Sensodetect AB, 222 22 Lund (SE)
(72) Inventor: HOLMBERG, Jens, S-254 38 Helsingborg (SE); KÄLLSTRAND, Johan, S-224 57 Lund (SE)
(74) Representative: AWA Sweden AB
(86) International application number: PCT/EP2014/050967
(87) International publication number: WO 2014/111554

(56) References cited:
- WO-A1-2008/043365
- WO-A2-2008/059289
- WO-A2-2012/028749
- NAM. DE BEER ET AL: "Haemodynamic responses to incision and sternotomy in relation to the auditory evoked potential and spontaneous EEG", BRITISH JOURNAL OF ANAESTHESIA, vol. 76, no. 5, 1 May 1996 (1996-05-01), pages 685-693, XP055105186, ISSN: 0007-0912, DOI: 10.1093/bja/76.5.685
- GUIGNARD ET AL: "Monitoring analgesia", BAILLIERE'S BEST PRACTICE AND RESEARCH, CLINICAL ANESTHESIOLOGY, BAILLIERE TINDALL, LONDON, US, vol. 20, no. 1, 1 March 2006 (2006-03-01), pages 161-180, XP005339064, ISSN: 1521-6896, DOI: 10.1016/J.BPA.2005.09.002
- PETOE MATTHEW ET AL: "On chirp stimuli and neural synchrony in the suprathreshold auditory brainstem response", THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, vol. 128, no. 1, 1 July 2010 (2010-07-01), pages 235-246, XP012135854, ISSN: 0001-4966, DOI: 10.1121/1.3436527

## Description

### BACKGROUND OF THE INVENTION

### Field of the Invention

This invention pertains in general to the field for monitoring depth of anaesthesia or awareness during general anaesthesia. Also, the invention pertains in general to the field for monitoring sensory functioning, such as transmission of pain pulses. In particular, the invention pertains to using Evoked Response Audiometry for monitoring depth of anaesthesia or monitoring sensory functioning.

### Description of the Prior Art

Awareness during general anaesthesia means that a patent is conscious during operation. This is a result of inadequate depth of anaesthesia. There are a number of different monitors on the market today for measuring depth of anaesthesia, but their reliability has been questioned.

WO2008/059289-A2, WO2008/043365-A1 and US2004/243017-A1 show different methods for monitoring anaesthetic depth and/or pain from auditory evoked neuron responses.

Hence, an improved system for monitoring depth of anaesthesia or detect awareness during general anaesthesia would be advantageous.

Further, the possibility of objectively monitoring sensory functions, such as transmission of pain pulses, to ensure that the sensory system, including the spinal cord, is functioning properly, would be advantageous.

Moreover, the ability of combining in one system both monitoring of depth of anaesthesia and monitoring of sensory functioning instead of relying on several systems or subjective methods, which is either complicated or uncertain, would be advantageous.

### SUMMARY OF THE INVENTION

Accordingly, examples of the present disclosure preferably seek to mitigate, alleviate or eliminate one or more deficiencies, disadvantages or issues in the art, such as the above-identified, singly or in any combination by providing an apparatus, a method, and a computer-readable medium, that may be used for monitoring sensory function of a subject and/or for monitoring depth of anaesthesia, according to the appended patent claims.

According to one aspect of the disclosure, an apparatus is provided which comprises a sound stimuli generating unit operative to send a sequence of an identical sound stimulus to a subject to evoke neurons response patterns. The apparatus also comprises a detection unit operative to detect a response signal of evoked neurons' response patterns related to each sound stimuli in the sequence. The apparatus further comprises a control unit operative to perform an analysis of the response signal of evoked neurons' response patterns.

Particularly, the disclosed apparatus may be used to measure or monitor specific sensory functioning using sound stimuli.

The apparatus may be adapted to perform monitoring of anaesthesia depth using audiometry. In some examples, the apparatus may be adapted to perform monitoring of sensory function of a subject using audiometry. In further examples, the apparatus is adapted to perform monitoring of anaesthesia depth and monitoring of sensory function of a subject simultaneously.

Particularly, the disclosed apparatus may be used to measure or monitor anaesthesia depth using sound stimuli.

In a further aspect of the disclosure, a method is provided comprising: generating a sequence of identical sound stimuli and transmitting the sound stimuli to a subject. Also, method comprises detecting a response signal of evoked neurons' response patterns related to each sound stimuli of the sequence. The method further comprises performing an analysis of the response signal of evoked neurons' response patterns.

The method may be adapted to perform monitoring of anaesthesia depth using audiometry. In some examples, the method may be adapted to perform monitoring of sensory function of a subject using audiometry. In further examples, the method may be adapted to perform monitoring of anaesthesia depth and monitoring of sensory function of a subject simultaneously.

According to a further aspect of the disclosure, a computer-readable medium having embodied thereon a computer program for processing by a computer is provided. The computer program comprising a plurality of code segments for carrying out the above described method and the method steps defined in the dependent claims.

In some examples, use of evoked response audiometry for monitoring depth of anaesthesia is disclosed.

In some further examples, use of evoked response audiometry for monitoring sensory functioning, such as transmission of pain pulses, is disclosed.

Also, in some examples, use of evoked response audiometry for monitoring sensory functioning, such as transmission of pain, and monitoring depth of anaesthesia simultaneously is disclosed

Further examples of the invention are defined in the dependent claims, wherein features for the second and subsequent aspects of the invention are as for the first aspect mutatis mutandis.

The term "one neuron" or "a group of neurons" refers herein to a neuron or a group of neurons belonging to the same cluster or structure, such as a nucleolus, interconnected nuclei or a layer. An evoked response from this one neuron or group of neurons may form a peak on a recorded response signal. If no response is evoked no peak may be formed or change in activity for that peak related to this one neuron or group of neurons.

It should be emphasized that the term "comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which examples of the invention are capable of will be apparent and elucidated from the following description of examples of the present discloser, reference being made to the accompanying drawings, in which
Fig. 1 is a graph that illustrates a typical brainstem response audiogram;
Fig. 2 is a schematic diagram of a device;
Fig. 3 is a flow chart that illustrates a method;
Fig. 4 is illustrating differences in amplitude between different stages of a procedure; and
Fig. 5A and 5B are illustrating measured data from two patients.

### DESCRIPTION OF THE PREFERRED EMBODIMENTS

Specific examples of the disclosure now will be described with reference to the accompanying drawings. This disclosure may, however, be embodied in many different forms and should not be construed as limited to the examples set forth herein; rather, these examples are provided so that this disclosure will be thorough and complete, and will fully convey the scope of the disclosure to those skilled in the art. The terminology used in the detailed description of the examples illustrated in the accompanying drawings is not intended to be limiting of the disclosure. In the drawings, like numbers refer to like elements.

The following description focuses on examples of the present disclosure applicable to an apparatus and method in the field of monitoring or measure depth of anaesthesia or awareness during general anaesthesia. Also, the description focuses on examples of the present disclosure applicable to an apparatus and a method in the field of monitoring or measuring sensory functioning, such as transmission of pain pulses.

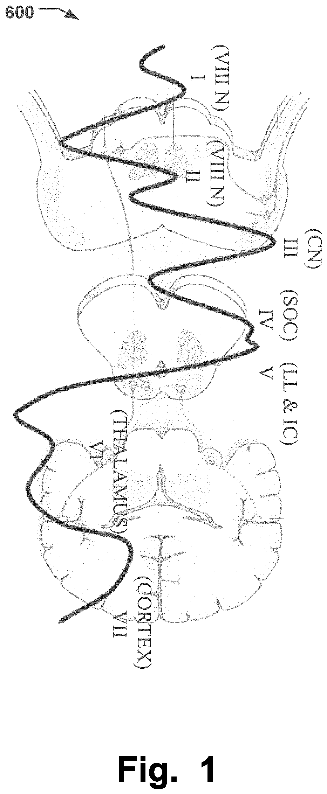

The latencies and amplitudes of the brainstem waves of Auditory Evoked Potentials (AEP) are called the Auditory Brainstem Response (ABR). ABR measures the electrical activity of the subcortical nerve cells generated by neuronal activity in the auditory pathways in the brainstem, within the first 10 milliseconds (ms) following acoustic stimulation. The wave pattern recorded consists of seven positive peaks, see Fig. 1, wave I is starting at the eight cranial nerve and extending into medulla (wave II). Wave III represents neuron activity at the level of auditory pons, and wave IV in the superior olivary complex (SOC). Wave V originate from the inferior colliculus, wave VI by the medial geniculate body of the thalamus and wave VII originate from the thalamocortical radiations.

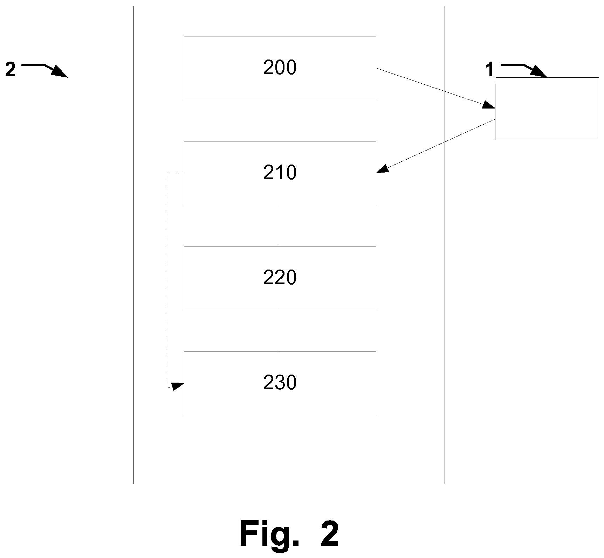

In an example an apparatus 2 is now described, which is illustrated with reference to Fig. 2. A sound stimuli generating unit 200 is operative to send a sequence of an identical sound stimulus to a subject to evoke neurons response patterns. The sound stimuli are transmitted to the ears of the subject 1, either to both ears or only to one ear.

Auditory stimuli used herein may be defined or referred to as "sound pulses" or signals, including but not limited to, transient peaks, clicks, tone bursts, or other suitable types of auditory stimuli suitable for repetitive presentation. The stimuli should be auditory perceptive to the subject to whom the stimuli is presented. Thus as defined within the area of psychoacoustics the sound stimuli presented to a human subject may have any frequencies between 20Hz and 20000 Hz, and the amplitude may be from the lower limit of audibility defined as 0 dB or higher but preferably below damaging level for the frequencies used. Preferably may be to use an amplitude being a minimum threshold amplitude or higher for the particular frequencies used, but below damaging level for the frequencies. Preferably the amplitude may be between about 0 to 120 dB, preferably between about 0 to 100 dB, preferably between about 0 to 90 dB, preferably about 70dB.

The time width or duration of each sound pulse may being the range of 0.1 to 1000ms. Preferably the time with or duration of each sound pulse may be approximately the time of the detection of the evoked response of the brainstem, thus preferably between 0 to 100 ms, such as 0 to 50ms, such as 0 to 10 ms.

An example of sound stimuli properties that have proven to have characteristics being particularly suited to be used when monitoring of depth of anaesthesia and/or for monitoring or measuring sensory functioning, such as transmission of pain pulses, is a square-shaped click tone. The sound stimuli may have a duration time of 0.1ms to 0.2ms, such as 0.12 to 0.15ms, such as 0.136ms. Additionally sound stimuli have a rise and fall time of 0.015ms to 0.03ms, such as 0.02ms to 0.025ms, such as 0.023ms.

Additionally and/or alternatively, the sound stimuli may be high pass filtered square pulses created by using a Fast Fourier Transformation-filter (FFT). The cut-off frequency between 1000Hz to 6000 Hz, and in particularly a cut-off such as 3000 Hz, or a cut-off such as 4000 Hz.

Also, for some example of the disclosure, the apparatus may generate and transmit sound stimuli with an interstimuli interval between individual stimulus in the range 100 to 250ms, such as 150 to 200 ms, such as 192 ms.

The apparatus comprises a detection unit 210 operative to detect a brainstem response signal related to the evoked neurons response patterns for each sound stimulus in the sequence. The response signals may be detected between 0 to 10ms, such as between 0-8ms, after the related sound stimulus has been generated and transmitted to the subject 1. In this ranges the relevant peaks for monitoring of depth of anaesthesia and/or monitoring of sensory functioning, such as transmission of pain pulses, can be found.

In some applications, the peaks in the range of 0 to 5ms may be of particular interest. In some applications, the peaks in the range of 4 to 8ms may be of particular interest. Thus in these cases, the apparatus may be restricted to a particular detection range. When narrowing the detection range, the analysis may be done faster since less data needs to be analysed.

The peaks that has shown to have the strongest response to applied stimuli to a sensory function, such as pain, during anaesthesia is activity in pons, Wave II and Wave III.

Detecting of the responses may be done by attaching electrodes to the skin of the subject's 1 head. For example, the electrodes may be attached at the mastoid bone, the forehead and behind the ears.

The apparatus may optionally comprise a storage unit 220 for storing the data for later use.

The apparatus 2, may also comprise a control unit 230 operative to perform an analysis of the response signal of evoked neurons' response patterns. The control unit 230 may comprise a computing unit, a processor or any other means for executing software code segments.

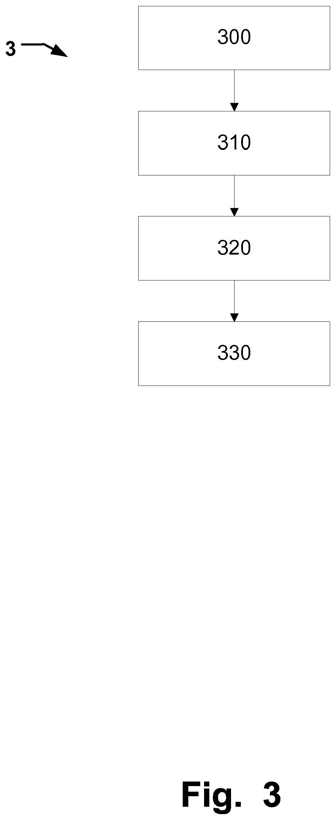

In Fig. 3, a flow-chart is used for illustrating a method of the disclosure. The method may be used for monitoring depth of anaesthesia or awareness during general anaesthesia. Additionally and/or alternatively fig.3 is illustrating a flow-chart for a method of monitoring or measuring sensory functioning.

The method starts by generating 300 a sequence of identical sound stimuli and transmitting the sound stimuli to a subject. The sound stimuli may evoke the subject's brainstem neurons. Each stimulus in the sequence may generate a corresponding response signal of evoked neurons' response patterns 310. The stimuli are the same as herein above described in relation to the apparatus of fig. 2.

All response signals of of evoked neurons' response patterns corresponding to each transmitted sound stimulus will be detected 320.

In the next step an analysis is performed 330 on the detected response signals of evoked neurons' response patterns.

For monitoring sensory functioning, the analysis may comprise, for example, detection of deviations by comparing detected responses of evoked neurons' response patterns to previous detected evoked neurons' response patterns from the subject. For example, every latest response signal following a stimulus is compared to each of the previously detected response signals. Alternatively, all response signals are detected and each individual response signal is compared to each of all detected response signals. Alternatively, all response signals are detected and each individual response signal is compared to an average calculated from all detected response signals.

In this way outlines may be excluded. Outlines may be brainstem responses having amplitudes which are divergent from a norm, such as the brainstem responses having the highest and/or the lowest amplitudes. The divergent or abnormal responses may be defined as a predetermined percentage of the total number of recorded responses having the highest and the lowest amplitude.

Alternatively, divergent or abnormal responses may be defined as brainstem responses having amplitudes that fall outside of a lower and an upper threshold.

Another, alternative may be to use a model, such as Pearson's correlation to remove responses which may have artifacts. Each response is than correlated against a normal audiogram. If the correlation coefficient is to low the response is excluded from further analysis.

These outlines may relate to artefacts, for instance breathing, coughs, and movements. To the remaining response signals, filters may be applied to remove specific artefacts related to cardiac activity, or the frequency of adjacent electrical equipment (50Hz). Different filters for removing these types of noise, such as a 50Hz noise, are readily available to the skilled person.

Additionally and/or alternatively, a profile matching of the detected response signals may be performed in the analysis. The profile matching ensures that, not only differences, but also characteristic aberration follows the patterns of an applied sensory functioning brainstem reactions.

If certain peaks in the brainstem responses are aberrant in amplitude, as compared to the same patient's reference values, and other peaks remains constant, a sensory functioning profile match was indicated. The patient's reference values may be obtained by transmitting sound stimuli to the patient before applying a test of a sensory functioning. Alternatively, the test of a sensory functioning may be done at any time during a transmission of a sequence of sound stimuli to the patient. When the test is applied the responses related the brainstem reaction to the applied test may be marked. One way of marking the responses is to time when the test is performed. In this way, when analysing the brainstem reaction, the responses related to the test may be separated from the other responses. This may be done because the time of the responses, related the applied test, in the sequence of sound stimuli is known. The other responses in the sequence may be used as reference values.

Alternatively and/or additionally, the comparison may be done to a database comprising recorded brainstem responses related to a single and/or a plurality of applied sensory functioning tests. Hence identify a sensory functioning profile match may be conducted. Thus a practitioner may be able to, objectively, ensure that the functioning of a sensory function is functioning properly.

One sensory functioning of special interest is transmission of pain pulses. Being able to objectively measuring the transmission of pain pulses would be an advantage within the field of health care.

The profile of a brainstem reaction to pain is complicated, thus does not only relate to higher single peak amplitudes. For example, brainstem reaction profiles generally may show increases in the amplitude of peak II and III, while other peaks may not show changes in the amplitude. In reaction to other applied sensory functioning tests, other peak combinations may increase while the rest of the peaks are constant.

Some reasons why transmission of pain pulses is of interest are the possibility for a practitioner to monitor that the neural pathway is not damaged during surgery, to objectively detect if a patient has damages to the neural pathway, such as damages to the spinal cord. It may also be used, during surgery, to detect if a patient may obtain perception of Phantom pain sensations after an amputation. This may be done by monitoring when a pain response reaction is no longer present in the evoke neurons response patterns of the brainstem. Alternatively and/or additionally, the method and apparatus may be used to detect if a patient suffer from Phantom pain sensations.

Additionally and/or alternatively, the apparatus or method may be used for measuring for how long the effect of pain may be perceived by monitoring the decline of arisen brainstem response reactions during, for example, the duration of a cut.

Other sensory functioning than transmission of pain pulses may be monitored, such as visual functioning, taste functioning, smell functioning or somatosensory functioning.

These analysis hereinabove described may be performed by the control unit in the apparatus illustrated in Fig. 2.

When monitoring depth of anaesthesia the same apparatus and method as hereinabove described may be used.

An exemplary way of performing monitoring of depth of anesthesia will now be described. Before the operation at least one baseline may be detected when the patient is awake, by generating and transmitting a sequence of sound stimuli to the subject. The baseline may be used during the analysis to minimize myogenic artifacts.

The patient will then be put under anesthesia. During the induction, during the anesthesia, and during operation, the patient may be monitored by either the apparatus described in relation to Fig. 2 or by the method described in relation to Fig. 3.

The brainstem response signals may be filtered to avoid artifacts and analyzed in respect to their amplitude.

One reason for testing the response signals to remove artifacts is to correlate them against averaged responses using Pearson's correlation. A high correlation value means that a response is relatively free from artifacts. Other methods may be used, such as the method hereinabove described.

Responses from different stages of the procedure, such as, pre-operation, during induction, when under anesthesia and during surgery may be compared and matched to detect deviations in the reposes.

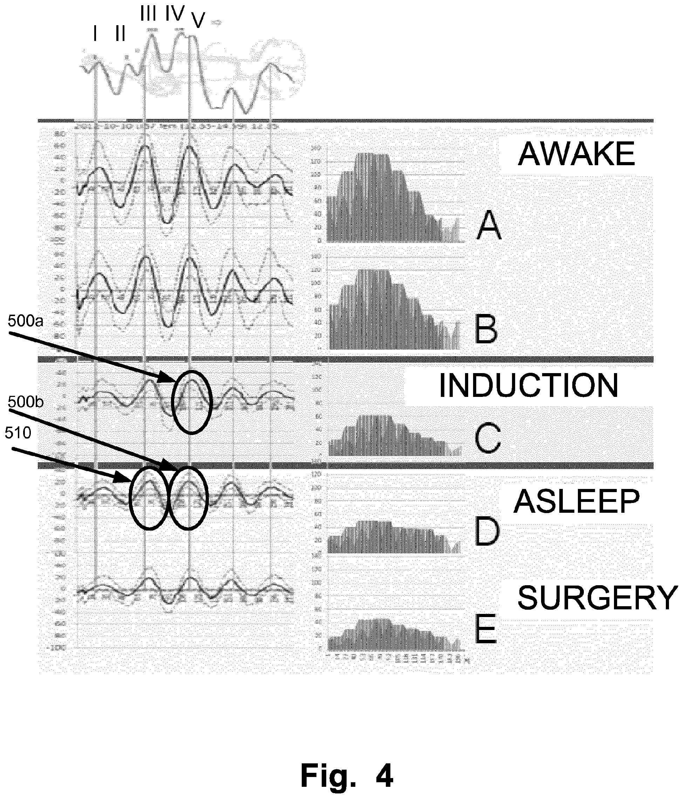

Fig. 4 is illustrating the amplitude of averaged response signals during different stages of the procedure.

It has been shown that even though all peaks are generally lower when the patient is under anesthesia. Peak III and peak V seems to have the most evident decrease in amplitude when comparing the stage when the patient is under anesthesia to the stage when the patient is awake.

These differences in amplitude are much more significant than when only detecting latencies.

An option is to use both amplitude and latencies for the monitoring.

According to the invention, monitoring depth of anaesthesia and monitoring a sensory functioning are performed simultaneously.

The potential of having the possibility to see both nerve function and anaesthesia depth in the same curve is substantial for the physician. If peak V is below a certain threshold, but peak III is raised in relation to stimulation of a sensory functioning, such as testing pain pulses by a needle pressure, the patient is satisfactorily in an aesthesia state as well as showing a functioning nervous system.

In Fig. 4 the patient is satisfactorily anesthetic due to the amplitude of peak V 500a,500b is lowered but the nervous system is working as a needle creates a profile change in Peak III 510.

When a patient is under anesthesia and since the anesthesia may be detected as an overall reduction of amplitudes in the measured brainstem response signal, a correlation between the depth of anaesthesia and the brainstem reaction seen in the response signals when applying test of a sensory functioning may be observed. This objective indication may be used by a practitioner, such as a surgeon, when deciding on how to perform a procedure. Alternatively, the practitioner may determine if the procedure is successful with no or little adverse effects for the patient.

The benefit for combining monitoring of anaesthesia depth with sensory function (such as transmission of pain pulses) is significant as the surgeon ensures that the sensory system of the patient, including the spinal cord, is functioning properly. Thus the device combining monitoring of anaesthesia depth with monitoring of sensory functioning has a greater value than having to rely on several systems (more complicated) or subjective methods (uncertain).

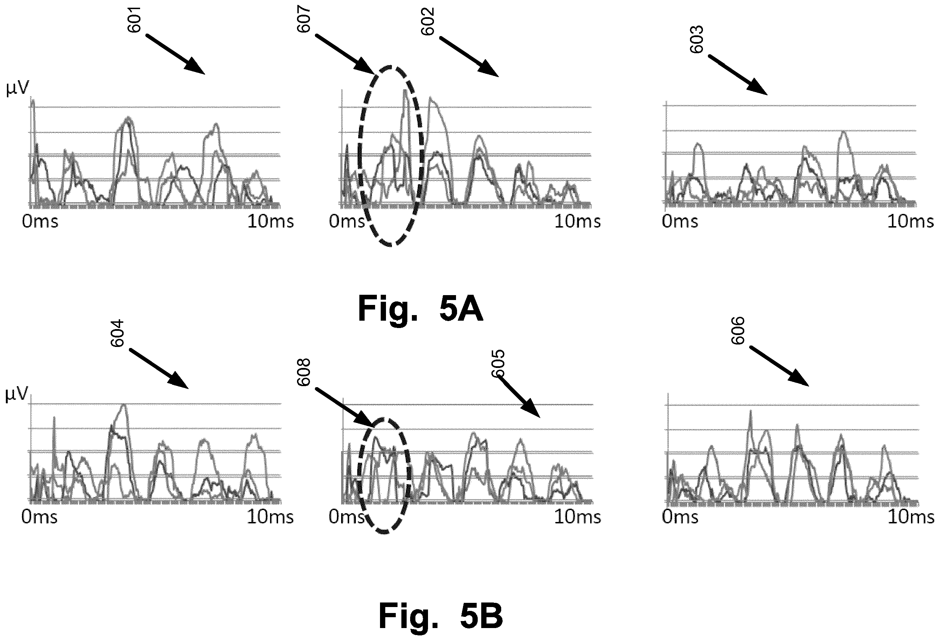

In figure 5A and 5B measured data from two patients are shown as an example, patient A in figure 5A and patient B in figure 5B. In each graph in figure 5A and figure 5B the different curves represents ABR recordings recorded every third second. This time can be altered.

In figure 5A graph 601 shows first measurements done when patient A is deeply sedated. Graph 602 shows second measurements when the patient A is deeply sedated a stung by a needle is a finger. Graph 603 shows third measurements done when patient A is deeply sedated again.

In the marked area 607 in graph 602 the activity in pons peak II and peak III is temporarily increased when the patient is stung compared to the first measurements in graph 601 done before being stung and the third measurements in graph 603 done after being stung.

In figure 5B graph 604 shows first measurements done when patient B is deeply sedated. Graph 605 shows second measurements when the patient B is deeply sedated a stung by a needle is a finger. Graph 606 shows third measurements done when patient B is deeply sedated again.

In the marked area 608 in graph 605 the activity in pons peak II and peak III is temporarily increased when the patient is stung compared to the first measurements in graph 604 done before being stung and the third measurements in graph 606 done after being stung.

This increase in the activity due to applied pain during anaesthesia which is shown in graph 602 in figure 5A and in graph 605 in figure 5B may be used to monitor or measure sensory functioning during sedation, in order to, for example, see that no nerves are damaged.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, i.e., elements that are conjunctively present in some cases and disjunctively present in other cases.

## Claims

1. A method for simultaneously monitoring or measuring, during operation, a sensory function and anaesthesia depth of a subject, said method comprising:
generating a sequence of identical sound stimuli and transmitting said sound stimuli to said subject;
detecting a response signal of evoked neurons' response patterns related to each sound stimuli of said sequence; and
performing an analysis of said sensory function and said anaesthesia depth based on said response signals of evoked neurons' response patterns detected when stimuli is applied to a sensory function compared to said response signals of evoked neurons' response patterns detected before and/or after stimuli was applied to said sensory function,
wherein said response signals are used both for the analysis of the sensory function as well as the anaesthesia depth, and
the sensory function is analyzed during operation to ensure that the sensory system is functioning.

2. The method according to claim 1, wherein said analysis comprising detecting deviations by comparing detected response of evoked neurons' response patterns to previous detected evoked neurons' response patterns from said subject; and/or, wherein said analysis further comprising performing a profile matching to detect characteristic aberration in amplitude compared to reference values of said subject; and/or, wherein said sound stimuli is generated using a Fast Fourier Transformation-filter; and/or, wherein said sound stimuli have a cut-off frequency between 1000 Hz to 6000 Hz, such as 3000 Hz, such as 4000 Hz; and/or, wherein said sound stimuli has a duration of 0.1ms to 0.2ms, such as 0.12 to 0.15ms, such as 0.136ms; and/or, wherein said sound stimuli have a rise and fall of 0.015ms to 0. 03ms, such as 0.02ms to 0.025ms, such as 0.023ms; and/or, wherein said detection of said response signal of evoked neurons' response patterns is done at 0-8ms, such as 0 to 5ms, such as 4 to 8ms, after said related sound stimulus.

3. An apparatus for simultaneously monitoring or measuring, during operation, a sensory function and anaesthesia depth of a subject, said apparatus comprising:
a sound stimuli generating unit (200) operative to send a sequence of an identical sound stimulus to said subject to evoke neurons response patterns;
a detection unit (210) operative to detect a response signal of evoked neurons' response patterns related to each sound stimuli in said sequence; and
a control unit (230) operative to perform an analysis of said sensory function and said anaesthesia depth based on said response signals of evoked neurons' response patterns detected when stimuli is applied to a sensory function compared to said response signals of evoked neurons' response patterns detected before and/or after stimuli was applied to said sensory function,
wherein said response signals are used both for the analysis of the sensory function as well as the anaesthesia depth, and
the sensory function is analyzed during operation to ensure that the sensory system is functioning.

4. The apparatus according to claim 3, wherein said control unit is operative to detect deviations by comparing detected response of evoked neurons' response patterns to previous detected evoked neurons' response patterns from said subject; and/or, wherein said control unit is further operative to perform a profile matching to detect characteristic aberration in amplitude compared to reference values of said subject.

5. The apparatus according to any of claims 3 to 4, wherein said sound stimuli is generated using a Fast Fourier Transformation-filter; and/or, wherein said sound stimuli have a cut-off frequency of between 1000 Hz to 6000 Hz, such as 3000 Hz, such as 4000 Hz; and/or, wherein said sound stimuli has a duration of 0.1ms to 0.2ms, such as 0.12 to 0.15ms, such as 0.136ms; and/or, wherein said sound stimuli have a rise and fall of 0. 015ms to 0. 03ms, such as 0.02ms to 0.025ms, such as 0. 023ms; and/or, wherein said monitored sensory functioning is transmission of pain pulses; and/or, wherein said detection of said response signal of evoked neurons' response patterns is done at 0-8ms, such as 0 to 5ms, such as 4 to 8ms, after said related sound stimulus.

6. A computer program comprising a plurality of code segments for carrying out of a method according to claims 1 to 2.

7. A computer-readable medium having embodied thereon a computer program according to claim 6.

## Patentansprüche

1. Verfahren zum gleichzeitigen Überwachen oder Messen einer sensorischen Funktion und Narkosetiefe eines Patienten während einer Operation, wobei das Verfahren umfasst:
Erzeugen einer Sequenz identischer Schallstimuli und Übertragen der Schallstimuli zu dem Patienten;
Detektieren eines Antwortsignals hervorgerufener Neuronen-Antwortmuster mit Bezug auf jeden Schallstimulus der Sequenz; und
Durchführen einer Analyse der sensorischen Funktion und der Narkosetiefe basierend auf den Antwortsignalen detektierter hervorgerufener Neuronen-Antwortmuster, wenn Stimuli an einer sensorischen Funktion beaufschlagt werden, im Vergleich zu den Antwortsignalen detektierter hervorgerufener Neuronen-Antwortmuster vor und/oder nach dem Beaufschlagen der sensorischen Funktion mit den Stimuli,
wobei die Antwortsignale für die Analyse sowohl der sensorischen Funktion wie auch der Narkosetiefe verwendet werden, und
die sensorische Funktion während der Operation analysiert wird, um sicherzustellen, dass das Sinnesorgan funktioniert.

2. Verfahren nach Anspruch 1, wobei die Analyse das Detektieren von Abweichungen mittels Vergleichs von detektierter Antwort von hervorgerufenen Neuronen-Antwortmustern auf zuvor detektierte hervorgerufene Neuronen-Antwortmuster von dem Patienten umfasst; und/oder wobei die Analyse ferner das Durchführen eines Profilabgleichs zum Detektieren charakteristischer Aberration bei der Amplitude im Vergleich zu Referenzwerten des Patienten umfasst; und/oder wobei die Schallstimuli unter Verwendung eines Fast-Fourier-Transformationsfilters erzeugt werden; und/oder wobei die Schallstimuli eine Grenzfrequenz zwischen 1000 Hz und 6000 Hz, wie z. B. 3000 Hz, wie z. B. 4000 Hz, aufweisen; und/oder wobei die Schallstimuli eine Dauer von 0,1 ms bis 0,2 ms, wie z. B. 0,12 ms bis 0,15 ms, wie z. B. 0,136 ms, aufweisen; und/oder wobei die Schallstimuli einen Anstieg und Abfall von 0,015 ms bis 0,03 ms, wie z. B. 0,02 ms bis 0,025 ms, wie z. B. 0,023 ms, aufweisen; und/oder wobei die Detektion des Antwortsignals hervorgerufener Neuronen-Antwortmuster bei 0-8 ms, wie z. B. 0 ms bis 5 ms, wie z. B. 4 ms bis 8 ms, nach dem diesbezüglichen Schallstimulus erfolgt.

3. Vorrichtung zum gleichzeitigen Überwachen oder Messen einer sensorischen Funktion und Narkosetiefe eines Patienten während der Operation, wobei die Vorrichtung umfasst:
eine Schallstimuli-Erzeugungseinheit (200), betreibbar zum Senden einer Sequenz eines identischen Schallstimulus zu dem Patienten, um Neuronen-Antwortmuster hervorzurufen;
eine Detektionseinheit (210), betreibbar zum Detektieren eines Antwortsignals hervorgerufener Neuronen-Antwortmuster mit Bezug auf jeden Schallstimulus in der Sequenz; und
eine Steuerungseinheit (230), betreibbar zum Durchführen einer Analyse der sensorischen Funktion und der Narkosetiefe, basierend auf den Antwortsignalen detektierter hervorgerufener Neuronen-Antwortmuster, wenn Stimuli an einer sensorischen Funktion beaufschlagt werden, im Vergleich zu den Antwortsignalen detektierter hervorgerufener Neuronen-Antwortmuster vor und/oder nach dem Beaufschlagen der sensorischen Funktion mit Stimuli,
wobei die Antwortsignale für die Analyse sowohl der sensorischen Funktion wie auch der Narkosetiefe verwendet werden, und
die sensorische Funktion während der Operation analysiert wird, um sicherzustellen, dass das Sinnesorgan funktioniert.

4. Vorrichtung nach Anspruch 3, wobei die Steuerungseinheit betreibbar ist, um Abweichungen mittels Vergleichs von detektierter Antwort von hervorgerufenen Neuronen-Antwortmustern mit zuvor detektierten hervorgerufenen Neuronen-Antwortmustern von dem Patienten zu detektieren; und/oder wobei die Steuerungseinheit ferner betreibbar ist zum Durchführen eines Profilabgleichs zum Detektieren charakteristischer Aberration bei der Amplitude im Vergleich zu Referenzwerten des Patienten.

5. Vorrichtung nach einem der Ansprüche 3 bis 4, wobei die Schallstimuli unter Verwendung eines Fast-Fourier-Transformationsfilters erzeugt werden; und/oder wobei die Schallstimuli eine Grenzfrequenz zwischen 1000 Hz und 6000 Hz, wie z. B. 3000 Hz, wie z. B. 4000 Hz, aufweisen; und/oder wobei die Schallstimuli eine Dauer von 0,1 ms bis 0,2 ms, wie z. B. 0,12 ms bis 0,15 ms, wie z. B. 0,136 ms, aufweisen; und/oder wobei die Schallstimuli einen Anstieg und Abfall von 0,015 ms bis 0,03 ms, wie z. B. 0,02 ms bis 0,025 ms, wie z. B. 0,023 ms, aufweisen; und/oder wobei die überwachte sensorische Funktionalität eine Übertragung von Schmerzimpulsen ist; und/oder wobei die Detektion des Antwortsignals hervorgerufener Neuronen-Antwortmuster bei 0-8 ms, wie z. B. 0 ms bis 5 ms, wie z. B. 4 ms bis 8 ms, nach dem diesbezüglichen Schallstimulus erfolgt.

6. Rechnerprogramm, umfassend eine Mehrzahl von Codesegmenten zum Durchführen eines Verfahrens nach den Ansprüchen 1 bis 2.

7. Rechnerlesbares Medium, aufweisend ein darauf vorliegendes Rechnerprogramm nach Anspruch 6.

## Revendications

1. Procédé permettant de surveiller ou de mesurer simultanément, pendant une opération, une fonction sensorielle et une profondeur d'anesthésie d'un sujet, ledit procédé comprenant les étapes consistant à :
générer une séquence de stimuli sonores identiques et transmettre lesdits stimuli sonores audit sujet ;
détecter un signal de réponse de schémas de réponse de neurones évoqués liés à chaque stimulus sonore de ladite séquence ; et
effectuer une analyse de ladite fonction sensorielle et de ladite profondeur d'anesthésie sur la base desdits signaux de réponse de schémas de réponse de neurones évoqués détectés lorsque des stimuli sont appliqués à une fonction sensorielle en comparaison avec lesdits signaux de réponse de schémas de réponse de neurones évoqués détectés avant et/ou après l'application de stimuli à ladite fonction sensorielle,
dans lequel
lesdits signaux de réponse sont utilisés à la fois pour l'analyse de la fonction sensorielle et de la profondeur d'anesthésie, et
la fonction sensorielle est analysée pendant l'opération pour assurer que le système sensoriel fonctionne.

2. Procédé selon la revendication 1, dans lequel ladite analyse comprend la détection d'écarts en comparant une réponse détectée de schémas de réponse de neurones évoqués à des schémas de réponse de neurones évoqués, détectés précédemment, dudit sujet ; et/ou dans lequel ladite analyse comprend en outre le fait d'effectuer un appariement de profils pour détecter une aberration caractéristique de l'amplitude en comparaison avec des valeurs de référence dudit sujet ; et/ou dans lequel lesdits stimuli sonores sont générés en utilisant un filtre à transformation de Fourier rapide ; et/ou dans lequel lesdits stimuli sonores présentent une fréquence de coupure entre 1000 Hz et 6000 Hz, comme 3000 Hz, comme 4000 Hz ; et/ou dans lequel, lesdits stimuli sonores ont une durée de 0,1 ms à 0,2 ms, comme 0,12 à 0,15 ms, comme 0,136 ms ; et/ou dans lequel lesdits stimuli sonores ont une montée et une descente de 0,015 ms à 0,03 ms, comme de 0,02 ms à 0,025 ms, comme 0,023 ms ; et/ou dans lequel, ladite détection dudit signal de réponse de schémas de réponse de neurones évoqués est effectuée entre 0 et 8 ms, comme entre 0 et 5 ms, comme entre 4 et 8 ms, après ledit stimulus sonore concerné.

3. Appareil permettant de surveiller ou de mesurer simultanément, pendant une opération, une fonction sensorielle et une profondeur d'anesthésie d'un sujet, ledit appareil comprenant :
une unité de génération de stimuli sonores (200) opérationnelle pour envoyer une séquence de stimuli sonores identiques audit sujet pour évoquer des schémas de réponse de neurones ;
une unité de détection (210) opérationnelle pour détecter un signal de réponse de schémas de réponse de neurones évoqués concernant chaque stimulus sonore dans ladite séquence ; et
une unité de commande (230) opérationnelle pour effectuer une analyse de ladite fonction sensorielle et de ladite profondeur d'anesthésie sur la base desdits signaux de réponse de schémas de réponse de neurones évoqués, détectés lorsque les stimuli sont appliqués à une fonction sensorielle en comparaison avec lesdits signaux de réponse de schémas de réponse de neurones évoqués, détectés avant et/ou après l'application à ladite fonction sensorielle,
dans lequel
lesdits signaux de réponse sont utilisés à la fois pour l'analyse de la fonction sensorielle et de la profondeur d'anesthésie, et
la fonction sensorielle est analysée pendant l'opération pour assurer que le système sensoriel fonctionne.

4. Appareil selon la revendication 3, dans lequel l'unité de commande est opérationnelle pour détecter des écarts en comparant une réponse détectée de schémas de réponse de neurones évoqués à des schémas de réponse de neurones évoqués, détectés précédemment, dudit sujet ; et/ou dans lequel ladite unité de commande est en outre opérationnelle pour effectuer un appariement de profils pour détecter une aberration caractéristique de l'amplitude en comparaison avec des valeurs de référence dudit sujet.

5. Appareil selon l'une quelconque des revendications 3 à 4, dans lequel lesdits stimuli sonores sont générés en utilisant un filtre à transformation de Fourier rapide ; et/ou dans lequel lesdits stimuli sonores présentent une fréquence de coupure entre 1000 Hz et 6000 Hz, comme 3000 Hz, comme 4000 Hz ; et/ou dans lequel, lesdits stimuli sonores ont une durée de 0,1 ms à 0,2 ms, comme de 0,12 à 0,15 ms, comme 0,136 ms ; et/ou dans lequel, lesdits stimuli sonores ont une montée et une descente de 0,015 ms à 0,03 ms, comme de 0,02 ms à 0,025 ms, comme 0,023 ms ; et/ou dans lequel, ladite détection dudit signal de réponse de schémas de réponse de neurones évoqués est effectuée entre 0 et 8 ms, comme entre 0 et 5 ms, comme entre 4 et 8 ms, après ledit stimulus sonore concerné.

6. Programme informatique comprenant une pluralité de segments de code pour effectuer un procédé selon les revendications 1 à 2.

7. Support lisible par ordinateur, sur lequel est incorporé un programme informatique selon la revendication 6.
